# EUROPÄISCHE PATENTSCHRIFT

(11) **EP 1 129 206 B1**
(45) Veröffentlichungstag und Bekanntmachung des Hinweises auf die Patenterteilung: **14.07.2004**
(21) Anmeldenummer: 99947431.5
(22) Anmeldetag: 28.09.1999
(51) Int. Cl.: C12P 1/00

(54) **GÄRUNGSRESTSTOFFAUFSCHLUSS**
FERMENTATION RESIDUE DECOMPOSITION
DISSOLUTION DE SUBSTANCES RESIDUELLES DE FERMENTATION

(30) Priorität: 02.10.1998 DE 19845508
(43) Veröffentlichungstag der Anmeldung: 05.09.2001
(73) Patentinhaber: Fraunhofer-Gesellschaft zur Förderung der angewandten Forschung e.V., 80686 München (DE)
(72) Erfinder: SCHOBER, Gabriele, D-70193 Stuttgart (DE); TRÖSCH, Walter, D-70329 Stuttgart (DE)
(86) Internationale Anmeldenummer: PCT/EP1999/007190
(87) Internationale Veröffentlichungsnummer: WO 2000/020618

(56) Entgegenhaltungen:
- WO-A-94/21394
- GB-A- 1 245 434
- US-A- 4 267 049
- US-A- 4 342 650
- DATABASE BIOSIS [Online] BIOSCIENCES INFORMATION SERVICE, PHILADELPHIA, PA, US GACESA, S. ET AL.: "Chaetomium cellulolyticum growht on substrates with anaerobic digested cattle and pig manure liquor" XP002135520
- DATABASE BIOSIS [Online] BIOSCIENCES INFORMATION SERVICE, PHILADELPHIA, PA, US MOO-YOUNG, M.: "pollution control of swine manure and straw by conversion to chaetomium-cellulolyticum single cell protein feed" XP002135521

## Beschreibung

Die vorliegende Erfindung betrifft ein Verfahren zum Aufschluß von Gärungsreststoffen, insbesondere zum Abbau von während einer Gärung gebildeten, einen wesentlichen Anteil anorganischer Substanzen enthaltenden Gärungsreststoffen.

Bekannt ist es, organische Abfallstoffe wie Klärschlämme, Biomüll, Restmüll oder organische Industrieabfälle zu Biogas zu vergären. Die dabei entstehenden Vergärungsreststoffe werden in vielen Fällen kompostiert, das heißt in fester Phase von Mikroorganismen abgebaut, oder zu Rekultivierungszwecken ausgebracht. Aufgrund der starken Schwermetallbelastung in den organischen Abfallstoffen wird es jedoch in zunehmendem Maße schwierig, den bei der Kompostierung anfallenden Kompost zu vermarkten. Wegen der rechtlichen Rahmenbedingungen ist es nicht möglich, Vergärungsreststoffe zu deponieren, da sie im allgemeinen einen organischen Trockensubstanzanteil von mehr als 5 % aufweisen. Auch die Verwertung als Sekundärwirtschaftsdünger wird an Bedeutung abnehmen, da die rechtlichen Rahmenbedingungen die aufbringbaren Applikationsmengen reduzieren. Schließlich ist es aus ökologischen und ökonomischen Gesichtspunkten heraus auch nicht sinnvoll, die Gärungsreststoffe zu verbrennen.

Es besteht also ein Bedarf, Gärungsreststoffe umweltverträglich, kostengünstig und in einem einfachen Verfahrensgang zu entsorgen oder abzubauen.

Bekannt ist es, daß bestimmte Pilze, insbesondere Weißfäulepilze, Lignin abbauen können. Bekannt ist es auch, daß Chaetomium cellulolyticum auf Weizenstroh, das heißt einem Lignocellulose-haltigen Material, kultiviert werden kann, wobei der Pilz die schlecht verwertbare Lignocellulose als Substrat nutzt und durch sein Wachstum diese mit proteinreicher Biomasse anreichert. Chaetomium cellulolyticum wird daher in der Futtermittelherstellung eingesetzt (Peiji G. et al. in Enzyme and Microbial Technology (20) 1997, 581-584; Tengerdy R. P. in "Solid Substrate Cultivation", Herausgeber Doelle H. W., Mitchell, D. A. und Rolz C. E., 1992). Schließlich ist es bekannt, daß thermisch und chemisch behandelter Schweinemist nach Vergärung mit Chaetomium cellulolyticum zum Abbau der Gärungsreststoffe kultiviert werden kann. Sowohl Weizenstroh als auch Schweinemist zeichnen sich jedoch durch einen sehr geringen Anteil an Schwermetallen und Salzen, das heißt anorganischen Substanzen, aus.

Bekannt ist, daß sich nur wenige halophile Organismen an Standorte hoher Salzkonzentrationen anpassen können. Es sind auch keine Verfahren durchgeführt oder bekanntgeworden, gemäß denen Pilze in Reinkultur oder Mischungen aus Reinkulturen zum Abbau von Gärungsreststoffen eingesetzt werden, die sich durch einen hohen Anteil an anorganischen Substanzen auszeichnen.

Das der vorliegenden Erfindung zugrundeliegende technische Problem besteht darin, ein umweltverträgliches, kostengünstiges und einfach durchzuführendes Verfahren zum Abbau von Gärungsreststoffen zur Verfügung zu stellen.

Die Erfindung löst dieses technische Problem durch die Bereitstellung eines Verfahrens zum Abbau von unvergorenen, anaerob nur schwer oder nicht abbaubaren Gärungsreststoffen, die einen hohen Anteil anorganischer Substanzen von mehr als 40 %, bezogen auf die Trockensubstanz, enthalten, wobei Reinkulturen oder Mischungen von Reinkulturen von Pilzen der Abteilung Eumycota mit den Gärungsreststoffen unter aeroben Bedingungen in einem wäßrigen Milieu kultiviert und die anorganischen Substanzen sowie CO₂ erhalten werden. Dabei werden erfindungsgemäß die anorganischen Substanzen in nahezu reiner oder hochkonzentrierter Form erhalten.

Die Erfindung stellt demgemäß ein Verfahren zum Abbau von Gärungsreststoffen zur Verfügung, die einen hohen Anteil anorganischer Substanzen von mehr als 40% enthalten. Im Zusammenhang mit der vorliegenden Erfindung wird unter einem Gärungsreststoff, der einen hohen Anteil anorganischer Substanzen von mehr als 40% enthält, ein Gärungsreststoff verstanden, der zu einem großen Teil aus anaerob nur schwer oder nicht abbaubaren Substanzen wie Lignocellulose, Pectinen, Huminstoffen, Cellulose, Zellwandbestandteilen und Bakterienbiomasse besteht und einen hohen Anteil an Schwermetallen, Schwermetallionen, anorganischen Salzen, Alkali, Erdalkali und Halogenidionen enthält, insbesondere Blei, Cadmium, Chrom, Kupfer, Nickel, Quecksilber, Zink, NaCl oder KCl sowie anorganischen Phosphat- und Stickstoffverbindungen. Der Anteil anorganischer Substanzen beträgt mehr als 40 %, insbesondere mehr als 45 %, besonders bevorzugt gleich oder mehr als 47 Gew.-%, bezogen auf Trockensubstanz an dem Gärungsreststoff. Die konkrete Zusammensetzung der Gärungsreststoffe richtet sich nach der Art und dem Edukt der vorangegangenen Gärung. Die Vergärung schließt eine Methan-Gärung mit ein. Es spielt erfindungsgemäß keine Rolle, welcher Art die Gärung und welcher Art das Edukt und das Produkt der Gärung war. Von Bedeutung für die vorliegende Erfindung ist es unter anderem vielmehr, daß die eingesetzten Gärungsreststoffe den beschriebenen hohen Anteil anorganischer Substanzen aufweisen. Überraschend ist nämlich, daß die erfindungsgemäß eingesetzten Pilze in Reinkultur oder Mischungen von Reinkulturen trotz des sehr hohen Anteils anorganischer Substanzen im Substrat, das heißt Gärungsreststoff-Substrat, dieses effizient und quantitativ zu CO₂ abbauen können.

Im Zusammenhang mit der vorliegenden Erfindung wird unter einer Kultivierung unter aeroben Bedingungen verstanden, daß die Kultivierung unter aktiver Belüftung stattfindet, insbesondere daß die Kultivierung unter Belüftung mit Druckluft, zum Beispiel aus der Umgebung, stattfindet.

Die Erfindung sieht auch in einer Ausgestaltung vor, daß die Kultivierung unter Mischen, vorzugsweise ständigem Mischen der Reaktionslösung, das heißt der in wäßrigem Milieu, also zum Beispiel in einer wäßrigen Lösung oder Suspension, vorhandenen Gärungsreststoffe und Pilze, stattfindet.

Die erfindungsgemäße Vorgehensweise, insbesondere die aerobe Kultivierung einer wäßrigen Reaktionslösung mit einem relativ niedrigen TS-(Trockensubstanz)Gehalt bis 30 % TS unter aktiver Luftzufuhr und Mischen durchzuführen, ermöglicht eine gegenüber herkömmlicher Kompostierung unerwartet hohe Abbaurate von Lignin und organischer Substanz.

In einer bevorzugten Ausführungsform der Erfindung ist vorgesehen, die Gärungsreststoffe vor der Kultivierung mit den Pilzen zu konditionieren oder zu hygienisieren. Überraschend ist auch, daß die Gärungsreststoffe keiner Sterilisierung unterzogen werden müssen, sondern vielmehr eine der Gärung nachgeschaltete Pasteurisierung oder eine zumindest teilweise thermophil durchgeführte Gärung ausreichen, um ein Wachstum der Pilze auf den einen hohen Anteil anorganischer Stoffe enthaltenden Gärungsreststoffen zu ermöglichen.

Die Erfindung sieht also in einer Ausgestaltung vor, daß die Gärungsreststoffe, bevor sie mit den Pilzen der Abteilung Eumycota kultiviert werden, konditioniert werden. Im Zusammenhang mit der vorliegenden Erfindung wird unter einem Konditionieren ein Vorgang verstanden, gemäß dem die Gärungsreststoffe so aufbereitet werden, daß diese ein bevorzugtes Wachstum der erfindungsgemäß eingesetzten aeroben Gärungsreststoffabbauer, das heißt der Pilze der Abteilung Eumycota, ermöglichen.

In einer bevorzugten Ausführungsform der Erfindung ist vorgesehen, die Konditionierung vorzunehmen, indem die Gärungsreststoffe vor der anschließenden Kultivierung mit den Pilzen der Abteilung Eumycota pasteurisiert werden. Die Pasteurisierung kann in einer besonders bevorzugten Ausführungsform der Erfindung durch Erhitzen auf 50°C bis 90°C°, insbesondere 60 bis 80°C, besonders bevorzugt 70°C, für 0,5 bis 3 Stunden, vorzugsweise 1 Stunde, durchgeführt werden.

In einer weiteren bevorzugten Ausführungsform der Erfindung kann vorgesehen sein, die Konditionierung durchzuführen, indem im Verlauf der Bildung der Gärungsreststoffe eine thermophile Gärung vorgesehen wird, insbesondere eine Gärung bei Temperaturen von 35 bis 70°C, insbesondere von 45 bis 65°C, besonders bevorzugt bei 55°C. Auf diese Weise wird erfindungsgemäß in vorteilhafter Weise erreicht, daß die für den Abbau mit den Pilzen der Abteilung Eumycota vorgesehenen Gärungsreststoffe bereits konditioniert sind, das heißt ein bevorzugtes Wachstum der erfindungsgemäß vorgesehenen Pilze stattfinden kann. Die Erfindung sieht in vorteilhafter Weise vor, die thermophile Gärung über eine hydraulische Verweilzeit von mindestens 24 Stunden durchzuführen.

Die Erfindung sieht, wie erwähnt, ferner in vorteilhafter Weise vor, die Kultivierung mit Reinkulturen oder Mischungen von Reinkulturen mit den Pilzen der Abteilung Eumycota aerob, insbesondere unter Belüftung, besonders bevorzugt unter Belüftung über Druckluft, durchzuführen, wobei eine ständige oder diskontinuierliche Luftzufuhr, zum Beispiel aus der Umgebung, mit einem Durchflußvolumen von 5-90, insbesondere 30-70, besonders bevorzugt 40 l/l Fermenter/h stattfindet .

Die Erfindung sieht in bevorzugter Weise vor, die aerobe Kultivierung mit Reinkulturen oder Mischungen von Reinkulturen mit den Pilzen der Abteilung Eumycota bei Temperaturen von 20 bis 42°C, insbesondere 37°C, und einem pH-Wert von 5,0 bis 8,5, bevorzugt 6,0 bis 7,0 in wäßriger Lösung durchzuführen. Vorteilhaft ist, daß der pH-Wert der Gärreststoffe nur geringfügig von 7,5, dem pH-Wert der Vergärung, herabgesetzt werden muß. In besonders bevorzugter Weise liegt der Trockensubstanzgehalt der in der wäßrigen Lösung vorliegenden Gärungsreststoffe bei 5 bis 30 %, insbesondere 10 bis 20 %, besonders bevorzugt bei 20 %.

Die Erfindung sieht ferner in einer weiteren bevorzugten Ausführungsform vor, daß die Pilze der Abteilung Eumycota Pilze der Klassen Basidiomycetes, insbesondere der Arten Phanerochaete chrysosporium, Phanerochaete BKM YM 125, Phanerochaete 1556, Pleurotus ostraetus, Pleurotus ostreatus var. florida frz., Pleurotus ostreatus var. florida holl., Trametes versicolor oder der Klasse Ascomycetes, insbesondere der Art Chaetomium cellulolyticum, sind.

Die Erfindung wird anhand der Beispiele und der dazugehörigen Figuren näher erläutert.

Die Figuren zeigen:
- Figur 1: den Verlauf des Organik-Gehaltes (ausgefüllte Quadrate), des DOC (offene Quadrate) und der CO₂-Bildung (ausgefüllte Kreise) während des Abbaus von Gärungsreststoffen durch Chaetomium cellulolyticum,
- Figur 2: die CO₂-Bildung (ausgefüllte Kreise) und die volumetrische CO₂-Bildungsrate (ausgefüllte Rauten) bei der Fermentation von Gärungsreststoffen durch Chaetomium cellulolyticum, kultiviert in wäßrigem Medium mit einem Trockensubstanzgehalt von 20 % und
- Figur 3: den Verlauf des oTS-Gehalts (ausgefüllte Quadrate), des DOC (offene Quadrate) und der CO₂-Bildung (ausgefüllte Kreise) während des Abbaus von Gärungsreststoffen durch Phanerochaete chrysosporium.

### Beispiel 1

### Gewinnung von Gärungsreststoffen

Als Ausgangsmaterial für die Vergärung wurden Küchenabfälle eines Hotels eingesetzt. Der eingesetzte Biomüll wurde weder chemisch, physikalisch oder thermisch vorbehandelt. Die Vergärung wurde in einer zweistufigen Vergärungsanlage mit zwei Reaktoren mit jeweils 30 1 Reaktionsvolumen durchgeführt. Die Gärreaktoren wurden mit frischem Klärschlamm einer kommunalen Kläranlage beschickt. Anschließend wurde der Biomüll zugefüttert und mit Wasser ein TS-Gehalt von < 20 % eingestellt.

Die Gärung (Methan-Gärung) wurde in einer ersten Stufe bei einer Temperatur von 37°C oder 55°C (alternativ) und einer zweiten Stufe mit einer Temperatur von 55°C durchgeführt. In beiden Reaktoren wurde kontinuierlich gerührt. Der pH-Wert in beiden Stufen wurde auf 7,3 bis 7,5 eingestellt. Der Abbaugrad des anaeroben Abbaus im Verlauf der zweistufigen Vergärung betrug 80 bis 90 %.

Nachdem der Vergärungsprozeß einige Monate stabil verlief, wurden die Gärungsreststoffe gesammelt und bei 5°C gelagert.

Die Gärungsreststoffe wurden nach einer Verweilzeit von 14 Tagen durch Zentrifugieren bei 12000 g auf 10 bis 20 % TS aufkonzentriert. Sie zeichnen sich durch einen hohen Anteil anorganischer Substanz, nämlich mindestens 40 bis 45 Gew.-% auf (TS) Trokkensubstanz aus. Der im Beispiel eingesetzte Gehalt anorganischer Substanzen betrug 47 % an dem gesamten Gärungsreststoff (a.TS).

In der folgenden Tabelle ist der Schwermetallgehalt der anorganischen Substanzen in den erfindungsgemäß eingesetzten Gärungsreststoffen aufgelistet.

**Tabelle 1**

| Schwermetall | Konzentration (mg/kg aTS) |
|---|---|
| Blei | 26 |
| Cadmium | < 5 |
| Chrom | 1199 |
| Kupfer | 339 |
| Nickel | 3197 |
| Quecksilber | 4 |
| Zink | 603 |

Die aufkonzentrierten Gärungsreststoffe wiesen ein Ammoniumgehalt von 1 bis 2 g/l auf. Der Gesamtstickstoffgehalt betrug ca. 5 % der Trockensubstanz (TS) beziehungsweise 10 % der oTS (organischen Trockensubstanz). Die Konzentration der löslichen anorganischen Substanzen betrug ca. 3 g/l.

Die so hergestellten Gärungsreststoffe wurden ohne weitere Behandlung oder Zugabe von weiteren Substanzen der aeroben Fermentation unterzogen.

### Beispiel 2

### Fermentation mit Reinkulturen oder Mischungen mit Reinkulturen von Ascomyceten, insbesondere Chaetomium cellulolyticum

Die in Beispiel 1 gewonnenen Gärungsreststoffe wurden in einen Fermenter gegeben und mit einer Reinkultur von Chaetomium cellulolyticum (ATCC 32319) angeimpft. Dabei wurde so angeimpft, daß die Biomasseanfangskonzentration 1 bis 2 g/l betrug. Die anschließende 28-tägige Fermentation wurde unter ständiger Druckluftzufuhr mit einem Durchflußvolumen von 40 l/l Fermenter/h (Umgebungsluft) und ständigem Durchmischen der Reaktionslösung aus Pilzen und Gärungsreststoffen durchgeführt.

Dabei wurde während der Fermentation die Trockensubstanz (TS)-Konzentration und die organische Trockensubstanz (oTS)-Konzentration ermittelt. Die Ermittlung erfolgte, indem Proben gewogen wurden und anschließend in einem Keramiktiegel über Nacht im Trockenschrank bei 105°C getrocknet wurden. Nach der Bestimmung der Gewichtsreduktion wurden die Proben 4 Stunden bei 500°C verascht und somit die aTS (anorganische Trockensubstanz)-Konzentration bestimmt. Der oTS-Gehalt berechnet sich aus der Gewichtsdifferenz zwischen TS und aTS. Der Gesamtstickstoffgehalt wurde nach der Methode von Kjeldahl bestimmt.

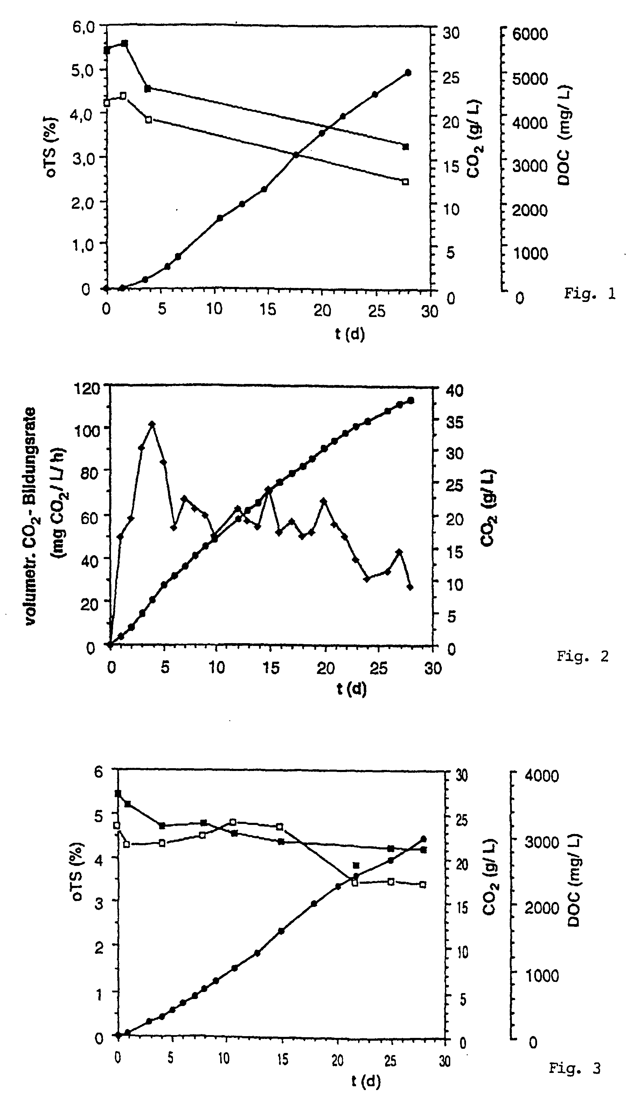

Der Figur 1 kann der Verlauf des Abbaus der Gärungsreststoffe entnommen werden. Dargestellt ist der Verlauf des Gehaltes organischer Stoffe, des DOC (dissolved organic carbons, Gehalt an gelöstem Kohlenstoff im Überstand) und der CO₂-Bildung während des Abbaus von Gärungsreststoffen durch Chaetomium cellulolyticum. Während der 28-tägigen Fermentation wurden durchschnittlich 0,76 g organischer Substanzen (oTS-Abbau) pro Liter und Tag abgebaut und 24,7 g/l CO₂ gebildet.

Der folgenden Tabelle 2 kann der Abbau einzelner Feststofffraktionen der Gärungsreststoffe nach 28 Tagen Fermentation durch Chaetomium cellulolyticum (10 % TS in wäßriger Lösung) entnommen werden.

**Tabelle 2**

| Fraktion | Konzentration Edukt (g/L) | Konzentration Produkt (g/L) | Abbau (%) |
|---|---|---|---|
| niedermolekulekulare wasserlösliche Substanzen | 17,1 | 11,4 | 33 |
| Lipide | 6,1 | 3,4 | 44 |
| Pectine, Hemicellulose, Phosphat | 33,9 | 26,3 | 22 |
| Cellulose | 8,0 | 4,5 | 43 |
| Lignin, Na-Humate | 9,9 | 5,5 | 45 |
| nicht lösliche oTS, Humine | 9,2 | 6,6 | 28 |

Chaetomium cellulolyticum wies mit 45 % eine sehr hohe Ligninabbaurate auf.

### Beispiel 3

### Variation des Trockensubstanz-Gehaltes bei dem Abbau durch Chaetomium cellulolyticum

Durch Variieren des Trockensubstanzgehaltes von 10 bis 75 % (in wäßriger Lösung) konnte festgestellt werden, daß eine besonders vorteilhafte Abbaurate mit Chaetomium cellulolyticum bei einem Trockensubstanzgehalt von ca. 20 Gew.-% erreicht werden kann.

Nach 28-tägiger Fermentation mit Chaetomium cellulolyticum bei einem Trockensubstanzgehalt von 20 % mit einem Anteil von 49 Gew.-% (auf Trockensubstanz) anorganischer Substanzen wurde ein durchschnittlicher Abbaugrad von 1,09 g organischer Substanzen pro Liter und Tag erreicht. Der Figur 2 kann der zeitliche Verlauf des dabei freigesetzten CO₂ entnommen werden.

Bei einem Trockensubstanzgehalt von 20 % ergibt sich ein oTS-Abbaurate von 1,09 g oTS/L/d bei einer CO₂-Bildung von 37,9 g/L.

### Beispiel 4

### Abbau von Gärungsreststoffen durch Basidiomyceten

Der Abbau von Gärungsreststoffen der in Beispiel 1 genannten Art wurde mit Phanerochaete chrysosporium BKM YM 125 bei 37°C und einem pH-Wert von 4,5 durchgeführt. Während des gesamten Versuchszeitraumes von 28 Tagen wurde die oTS um 22 % und der DOC um 27 % reduziert. Dabei wurden 22,4 g CO₂/l produziert. Die Ergebnisse sind graphisch in Figur 3 dargestellt. Die Kultivierungsbedingungen entsprachen den in Beispiel 2 beschriebenen, falls nicht anders angegeben.

Der folgenden Tabelle 3 kann entnommen werden, daß weitere Basidiomyceten zum Abbau von Gärungsreststoffen eingesetzt werden können.

**Tabelle 3**

| Species | oTS-Reduktion |
|---|---|
| Phanerochaete | 30 % |
| chrysosporium | 21 % |
| Phanerochaete | 24 % |
| BKM YM 125 | 18 % |
| Phanerochaete 1556 | 26 % |
| | 20 % |
| Pleurotus ostraetus | 21 % |
| | 9 % |
| Pleurotus ostreatus var. | 21 % |
| florida frz. | 25 % |
| Pleurotus ostreatus var. | 14 % |
| florida holl. | 26 % |
| Trametes versicolor | 16 % |
| | 20 % |

Dargestellt ist die Reduktion der oTS nach 4 Wochen Inkubation.

## Patentansprüche

1. Verfahren zum Abbau von anaerob nur schwer oder nicht abbaubaren Gärungsreststoffen, die einen hohen Anteil anorganischer Substanzen von mehr als 40%, bezogen auf die Trockensubstanz, enthalten, wobei Reinkulturen oder Mischungen aus Reinkulturen von Pilzen der Abteilung Eumycota mit den Gärungsreststoffen unter aeroben Bedingungen in einem wässrigen Milieu kultiviert und die anorganischen Substanzen sowie CO₂ erhalten werden.

2. Verfahren nach Anspruch 1, wobei die Gärungsreststoffe vor der Kultivierung konditioniert werden.

3. Verfahren nach Anspruch 2, wobei die Konditionierung in Form einer Pasteurisierung über einen Zeitraum von 0,5 bis 3 Stunden, insbesondere 1 Stunde, bei einer Temperatur von 50 bis 90°C, insbesondere 70°C, durchgeführt wird.

4. Verfahren nach Anspruch 2, wobei die Konditionierung während der durch Gärung erfolgten Bildung der unvergärbaren Gärungsreststoffe erfolgt, insbesondere bei Temperaturen zwischen 35 und 65°C.

5. Verfahren nach einem der vorhergehenden Ansprüche, wobei die Gärung bei einem pH-Wert zwischen 5,0 und 8,5 durchgeführt wird.

6. Verfahren nach einem der vorhergehenden Ansprüche, wobei die anorganischen Substanzen Schwermetalle, Schwermetallionen, Alkali-, Erdalkali- und Halogenidionen sowie anorganische Salze sind, insbesondere Blei, Cadmium, Chrom, Kupfer, Nickel, Quecksilber, Zink, NaCl und/oder KCl sowie anorganische Phosphat- und Stickstoffverbindungen.

7. Verfahren nach einem der vorhergehenden Ansprüche, wobei die Pilze der Abteilung Eumycota Basidiomyceten oder Ascomyceten sind.

8. Verfahren nach einem der vorhergehenden Ansprüche, wobei die Pilze der Abteilung Eumycota aus der Art Chaetomium cellulolyticum stammen.

9. Verfahren nach einem der vorhergehenden Ansprüche, wobei die Kultivierung in einer wäßrigen Lösung mit einem Trockensubstanzgehalt von 5 bis 30 Gew.-%, insbesondere 10 bis 20 Gew.-%, erfolgt.

10. Verfahren nach einem der vorhergehenden Ansprüche, wobei die Kultivierung unter Zufuhr von Luft, insbesondere Umgebungsluft, stattfindet.

11. Verfahren nach Anspruch 10, wobei die Zufuhr von Luft mit einem Durchflußvolumen von 5 - 90 l/l Fermenter/h, bevorzugt 30-70 l/l Fermenter/h, besonders bevorzugt 40 l/l Fermenter/h stattfindet.

12. Verfahren nach einem der vorhergehenden Ansprüche, wobei die Kultivierung unter Mischen stattfindet.

## Claims

1. Method for fragmenting fermentation residues which are difficult or impossible to fragment anaerobically and which contain a high proportion of inorganic substances of more than 40%, relative to the dry substance, wherein pure cultures or mixtures of pure cultures of fungi from the division Eumycota are cultivated with the fermentation residues under aerobic conditions in an aqueous medium and the inorganic substances and CO₂ are obtained.

2. Method according to claim 1, wherein the fermentation residues are conditioned before being cultivated.

3. Method according to claim 2, wherein the conditioning is carried out in the form of pasteurisation over a period of between 0.5 and 3 hours, especially 1 hour, at a temperature of 50 to 90°C, especially 70°C.

4. Method according to claim 2, wherein the conditioning takes place during the formation of the unfermentable fermentation residues due to fermentation, especially at temperatures between 35 and 65°C.

5. Method according to one of the preceding claims, wherein the fermentation takes place at a pH value of between 5.0 and 8.5.

6. Method according to one of the preceding claims, wherein the inorganic substances are heavy metals, heavy metal ions, alkali ions, earth alkali ions and halide ions as well as inorganic salts, especially lead, cadmium, chromium, copper, nickel, mercury, zinc, NaCl and/or KCl and inorganic phosphate and nitrogen compounds.

7. Method according to one of the preceding claims, wherein the fungi of the division Eumycota are Basidiomycetes or Ascomycetes.

8. Method according to one of the preceding claims, wherein the fungi of the division Eumycota originate from the species Chaetomium cellulolyticum.

9. Method according to one of the preceding claims, wherein the cultivation takes place in an aqueous solution with a dry substance content of 5 to 30 % by weight, especially 10 to 20 % by weight.

10. Method according to one of the preceding claims, wherein the cultivation takes place with a supply of air, especially ambient air.

11. Method according to claim 10, wherein the air is supplied at a flow volume of 5-90 l/l fermenter/h, preferably 30-70 l/l fermenter/h, by particular preference 40 l/l fermenter/h.

12. Method according to one of the preceding claims, wherein the cultivation takes place with mixing.

## Revendications

1. Procédé pour la dégradation de résidus de fermentation seulement difficilement ou non dégradables par voie anaérobie, qui ont une forte teneur en substances non organiques de plus de 40 % par rapport à la matière sèche,
**caractérisé en ce que**
dans un milieu aqueux avec les résidus de fermentation, dans des conditions aérobies, on cultive des cultures pures ou des mélanges de cultures pures de champignons appartenant à l'embranchement de « Eumycota », et on obtient les substances non organiques ainsi que du CO₂.

2. Procédé selon la revendication 1,
**caractérisé en ce que**
les résidus de fermentation sont conditionnés avant la culture.

3. Procédé selon la revendication 2,
**caractérisé en ce que**
le conditionnement est effectué sous forme d'une pasteurisation en un espace de temps de 0,5 à 3 heures, en particulier de 1 heure, à une température de 50 à 90°C, en particulier de 70°C.

4. Procédé selon la revendication 2,
**caractérisé en ce que**
le conditionnement s'effectue pendant la formation, effectuée par fermentation, des résidus non fermentescibles de fermentation, en particulier à des températures comprises entre 35 et 65°C.

5. Procédé selon l'une quelconque des revendications précédentes,
**caractérisé en ce que**
la fermentation est effectuée à un pH compris entre 5,0 et 8,5.

6. Procédé selon l'une quelconque des revendications précédentes,
**caractérisé en ce que**
les substances non organiques sont des métaux lourds, des ions de métaux lourds, des ions alcalins, alcalino-terreux et halogénures, ainsi que des sels minéraux, en particulier le plomb, le cadmium, le chrome, le cuivre, le nickel, le mercure, le zinc, NaCl et/ou KCl, ainsi que des phosphates et des composés azotés non organiques.

7. Procédé selon l'une quelconque des revendications précédentes,
**caractérisé en ce que**
les champignons appartenant à l'embranchement de « Eumycota » sont des basidiomycètes ou des ascomycètes.

8. Procédé selon l'une quelconque des revendications précédentes,
**caractérisé en ce que**
les champignons appartenant à l'embranchement de « Eumycota » sont issus de l'espèce *Chaetonium cellulolyticum*.

9. Procédé selon l'une quelconque des revendications précédentes,
**caractérisé en ce que**
la culture s'effectue dans une solution aqueuse ayant une teneur en matière sèche de 5 à 30 % en poids, en particulier de 10 à 20 % en poids.

10. Procédé selon l'une quelconque des revendications précédentes,
**caractérisé en ce que**
la culture a lieu avec apport d'air, en particulier d'air ambiant.

11. Procédé selon la revendication 10,
**caractérisé en ce que**
l'apport d'air a lieu à un volume de flux de 5 - 90 l/l de fermenteur/h, de préférence de 30 - 70 l/l de fermenteur/h, de façon particulièrement préférée de 40 l/l de fermenteur/h.

12. Procédé selon l'une quelconque des revendications précédentes,
**caractérisé en ce que**
la culture a lieu avec mélange.
